(19) Europäisches Patentamt
European Patent Office
Office européen des brevets

(11) EP 0 908 172 A1

(12) **EUROPÄISCHE PATENTANMELDUNG**

(43) Veröffentlichungstag:
**14.04.1999 Patentblatt 1999/15**

(51) Int. Cl.$^6$: **A61K 7/42**

(21) Anmeldenummer: **98109941.9**

(22) Anmeldetag: **30.05.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **13.06.1997 DE 19725087**

(71) Anmelder:
**Beiersdorf Aktiengesellschaft**
**D-20253 Hamburg (DE)**

(72) Erfinder:
- **Gers-Barlag, Heinrich, Dr.**
**25495 Kummerfeld (DE)**
- **Kröpke, Rainer**
**22527 Hamburg (DE)**

(54) **Kosmetische und dermatologische Lichtschutzformulierungen in Form von O/W Emulsionen mit einem Gehalt an hydrophoben anorganischen Mikropigmenten und hydrophilen Tensiden**

(57) O/W-Emulsionen,

- enthaltend eine Wasserphase, gegebenenfalls übliche, in Wasser lösliche oder dispergierbare Substanzen,
- enthaltend eine Ölphase, in welcher ein oder mehrere anorganische Pigmente, insbesondere hydrophobe anorganische Mikropigmente, in suspendierter Form vorliegen,
- enthaltend mindestens ein hydrophiles Tensid.

EP 0 908 172 A1

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasem des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nicht-radikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Solche Pigmente werden häufig einer Obeflächen behandlung unterzogen, dergestalt, daß entweder durch kovalent gebundene hydrophobe Molekülgruppen oder durch bloße Adsorption hydrophober Moleküle an der Oberfläche einer Pigmentpartikel eine Hydrophobierung erzielt wird, die zum Zwecke hat, die Dispergierbarkeit in Lipiden zu verbessern.

Die anorganischen Pigmente zeichnen sich an sich durch gute Lichtschutzwirkung aus. Sie haben jedoch den Nachteil, daß es schwierig ist, sie in befriedigender Weise solchen Formulierungen einzuverleiben. Nur wenn die Partikel in der endgültigen Formulierung sehr klein sind, werden sie nach dem Auftragen auf die Haut nicht als störendes „Weißeln" (Bildung von weißen Flecken auf der Haut) beobachtet. Üblicherweise liegen die Partikelgrößen solcher Pigmente im Bereich unter 100 nm. In einer herkömmlichen Emulsion neigen die Partikel mehr oder weniger stark zur Zusammenlagerung zu Agglomeraten, welche bereits unter dem Lichtmikroskop zu erkennen sind. Solche Agglomeration ist ferner nicht mit dem Herstellungsprozeß einer entsprechenden Zubereitung abgeschlossen, sondern setzt sich während der Lagerung fort. Das „Weißeln" kann sich daher über einen längeren Zeitraum noch verstärken.

Ein weiterer Nachteil ist, daß es in entsprechenden Emulsionen, insbesondere O/W-Emulsionen, zu Migrationen anorganischer Partikel zwischen der Ölphase, wo ihre Gegenwart erwünscht ist, und der Wasserphase kommen kann. Finden sich aber in der Wasserphase derartige Partikel als Kristallisationskeime, so daß bestimmte Stoffe, unter anderem Emulgatoren, und von diesen insbesondere die Stearinsäure, auskristallisieren und dadurch die Stabilität der Emulsionen stark beeinträchtigen.

Auch kann das Ausölen oder gar Brechen einer Emulsion mittel- oder langfristig Folge einer derartigen Agglomeration sein.

Verschlimmernd kommt hinzu, daß sich auch die UV-Absorptions- bzw. UV-Reflexionseigenschaften einer Zubereitung verschlechtern, da diese Eigenschaften stark abhängig sind von der Dispersität der Partikel.

Ein weiterer Nachteil des Einsatzes anorganischer Pigmente in kosmetischen Formulierungen ist, daß solche Pigmente in den weitaus meisten Fällen zu starker Hauttrockenheit führen.

Aufgabe der vorliegenden Erfindung war mithin, den Nachteilen des Standes der Technik abzuhelfen.

Es hat sich überraschenderweise gezeigt, und darin liegt die Lösung der Aufgaben begründet, daß O/W-Emulsionen,

- enthaltend eine Wasserphase, gegebenenfalls übliche, in Wasser lösliche oder dispergierbare Substanzen,
- enthaltend eine Ölphase, in welcher ein oder mehrere anorganische Pigmente, insbesondere hydrophobe anorganische Mikropigmente, in suspendierter Form vorliegen,
- enthaltend mindestens ein hydrophiles Tensid

den Nachteilen des Standes der Technik abhelfen.

Erfindungsgemäß ist auch die Verwendung von hydophilen Tensiden in O/W-Emulsionen, diese

- enthaltend eine Wasserphase, gegebenenfalls übliche, in Wasser lösliche oder dispergierbare Substanzen,
- enthaltend eine Ölphase, in welcher ein oder mehrere anorganische Pigmente, insbesondere hydrophobe anorganische Mikropigmente, in suspendierter Form vorliegen,

zur Stabilisierung der O/W-Emulsion gegen die Migration der anorganischen Pigmente, insbesondere hydrophoben anorganischen Mikropigmente aus der Ölphase in die Wasserphase und die Folgen einer solchen Migration.

Erfindungsgemäß ist ferner auch die Verwendung von hydophilen Tensiden in O/W-Emulsionen, diese

- enthaltend eine Wasserphase, gegebenenfalls übliche, in Wasser lösliche oder dispergierbare Substanzen,
- enthaltend eine Ölphase, in welcher ein oder mehrere anorganische Pigmente, insbesondere hydrophobe anorganische Mikropigmente, in suspendierter Form vorliegen,

zur Stabilisierung der O/W-Emulsionen gegen das Weißeln der anorganischen Pigmente, insbesondere der hydrophoben anorganischen Mikropigmente.

Erfindungsgemäß verwendete anorganische Pigmente werden insbesondere vorteilhaft gewählt aus der Gruppe der oberflächlich hydrophobierten Metalloxide und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Erfindungsgemäß liegen die anorganischen Pigmente in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, UVITAN 160 und UVITAN 161 von der Firma Kemira sowie T805 von der Firma Degussa erhältlich.

Die Gesamtmenge an hydrophoben anorganischen Mikropigmenten in den fertigen kosmetischen oder dermatologischen Zubereitungen wird vorteilhatt aus dem Bereich von 0,1 - 20,0 Gew.-%, bevorzugt 0,5 - 10,0 Gew.-% gewählt, bezogen auf das Gesamtgewicht der Zubereitungen.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

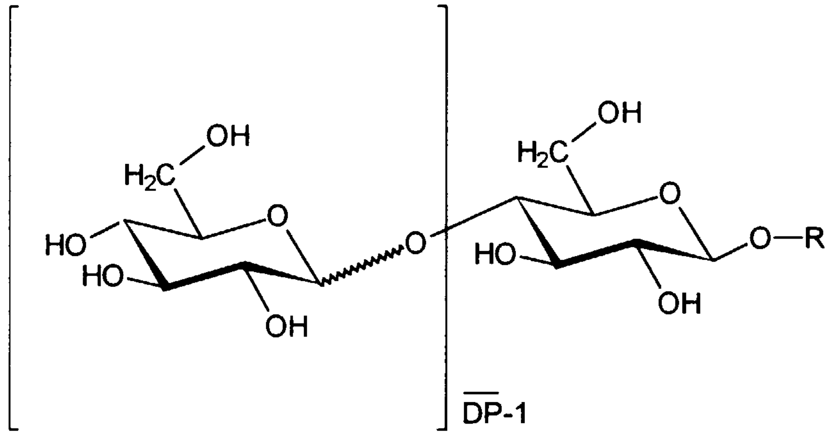

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum_i \frac{p_i}{100} \cdot i$$

Dabei stellen $p_1$, $p_2$, $p_3$ ... bzw, $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1 - 2, insbesondere vorteilhaft von 1,1 bis 1,5, ganz besonders vorteilhaft von ungefähr 1,3 gewählt.

Der Wert DP trägt dem Umstande Rechnung, daß Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40 - 70 Gew.-%.

Erfindungsgemäß besonders vorteilhaft verwendete Alkylglycoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

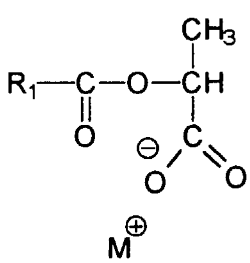

auszeichnen, wobei $R_1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der

Gesellschaft American Ingredients Company.

Die Betaine werden vorteilhaft gewählt aus der Gruppe der Substanzen, welche sich durch die Strukturformel

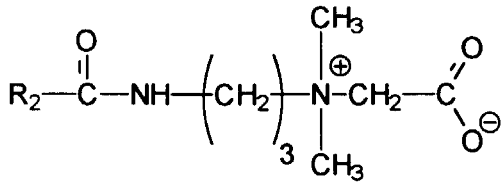

auszeichnen, wobei $R_2$ einen verzweigten oder unverzweigten Alkylrest mit 1 bis 30 Kohlenstoffatomen bedeutet.

Insbesondere vorteilhaft bedeutet $R_2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Emulsionen sind vorteilhaft dadurch gekennzeichnet, daß das oder die hydrophilen Tenside in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

Es ist vorteilhaft, weitere übliche Co-Emulgatoren in den erfindungsgemäßen O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11 - 16, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth19), Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16),

Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.
Als Alkylethersulfat kann Natrium Laureth 1-4 sulfat vorteilhaft verwendet werden.
Als ethoxyliertes Cholesterinderivat kann vorteilhaft Polyethylenglycol(30)Cholesterylether verwendet werden. Auch Polyethylenglycol(25)Sojasterol hat sich bewährt.
Als ethoxylierte Triglyceride können vorteilhaft die Polyethylenglycol(60) Evening Primrose Glycerides verwendet werden (Evening Primrose = Nachtkerze)
Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.
Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.
Als fakultative, dennoch erfindungsgemäß gegebenenfalls vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.
Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.
Es ist erfindungsgemäß vorteilhaft, in den erfindungsgemäßen Zubereitungen übliche öllösliche UVA-Filter und/oder UVB-Filter in der Lipidphase und/oder übliche wasserlösliche UVA-Filter und/oder UVB-Filter in der wäßrigen Phase einzusetzen.
Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.
Die UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Ein vorteilhafter UVB-Filter, der vorteilhaft im Sinne der vorliegenden Erfindung verwendet werden kann, ist der 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin.

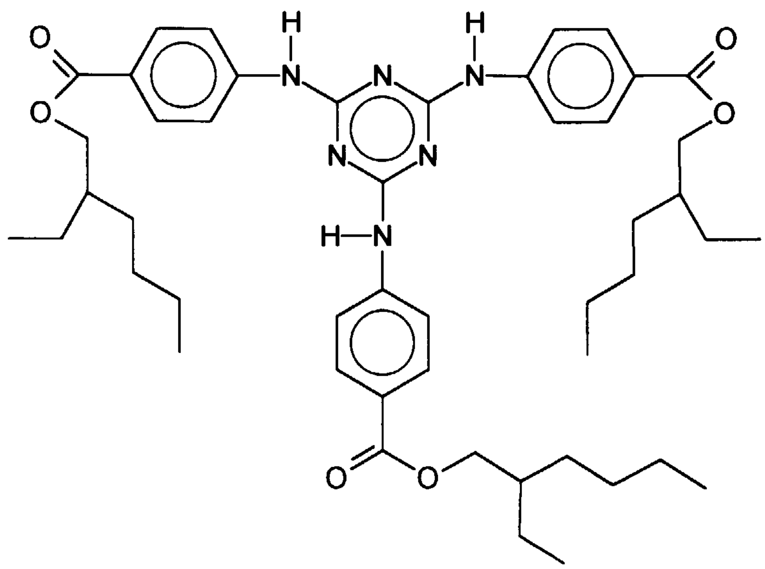

Diese UVB-Filtersubstanz wird von der BASF Aktiengesellschaft unter der Warenbezeichnung UVINUL® T 150 vertrieben und zeichnet sich durch gute UV-Absorptionseigenschaften aus.

Besonders vorteilhaft sind erfindungsgemäße Zubereitungen, welche als weitere UV-Schutzsubstanzen Salicylsäurederivate 4-Isopropylbenzylsalicylat, 2-Ethylhexylsalicylat, (Octylsalicylat) und/oder Homomenthylsalicylat enthalten, insbesondere solcher Salicylsäurederivate der allgemeinen Struktur

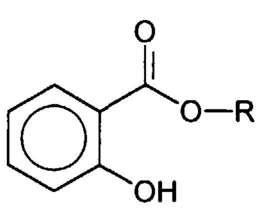

wobei R darstellen kann:

(1) einen verzweigten oder unverzweigten Alkylrest mit 5 - 18 Kohlenstoffatomen oder

(2) einen unsubstituierten Cycloalkylrest mit 5 - 7 Kohlenstoffatomen oder

(3) einen mit bis zu 10 verzweigten oder unverzweigten Alkylresten mit 5 - 18 Kohlenstoffatomen substituierten Cycloalkylrest mit 5 - 7 Kohlenstoffatomen oder

(4) einen unsubstituierten Phenylrest oder

(5) einen mit bis zu 5 verzweigten oder unverzweigten Alkylresten mit 5 - 18 Kohlenstoffatomen substituierten Phenylrest.

Zu gebräuchlichen UV-Filtern gehören darunter

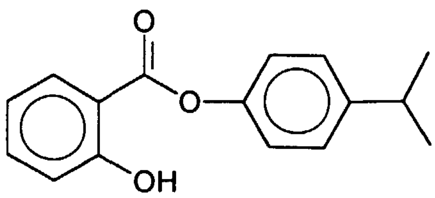

**(4-Isopropylbenzylsalicylat),**

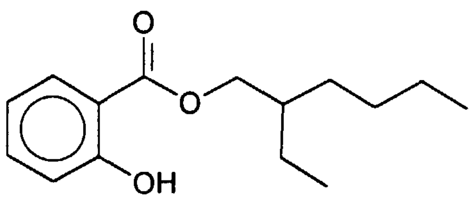

**(2-Ethylhexylsalicylat, Octylsalicylat),**

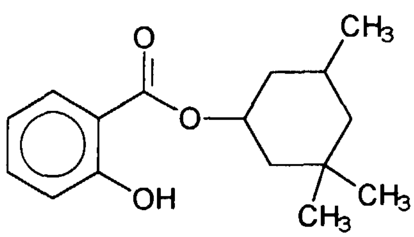

**(Homomenthylsalicylat).**

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter in den erfindungsgemäßen Zubereitungen einzusetzen, welche bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder

mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, nButylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewährt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexlisostearat, Octyldodecanol, Isotridecylisononanoat, Isoeicosan, 2-Ethylhexlcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen das Wesen der vorliegenden Erfindung näher umreißen, ohne die Erfindung einzuschränken.

| Beispiel 1<br>O/W-Lotion | |
|---|---|
| Glycerylstearat SE | 3,50 |
| Stearinsäure | 1,80 |
| Glycerin | 3,00 |
| Cetylsteaylalkohol | 0,50 |
| Octyldodecanol | 7,00 |
| Dicaprylylether | 8,00 |
| Cetylstearylisononanoat | 6,00 |
| Plantaren® 2000 | 1,00 |

(fortgesetzt)

| Beispiel 1<br>O/W-Lotion | |
|---|---|
| hydrophobes $TiO_2$ | 1,00 |
| Carbomer | 0,20 |
| NaOH (45%-ig) | 0,20 |
| Konservierungsmittel | q.s. |
| Parfum | q.s. |
| Wasser, demin. | ad 100,00 |

| Beispiel 2<br>O/W-Lotion | |
|---|---|
| Glycerylstearat SE | 3,50 |
| Stearinsäure | 1,80 |
| Glycerin | 3,00 |
| Cetylstearylalkohol | 0,50 |
| Octyldodecanol | 7,00 |
| Dicaprylylether | 8,00 |
| Cetylstearylisononanoat | 6,00 |
| Plantaren® 2000 | 1,00 |
| hydrophobes ZnO | 1,00 |
| Carbomer | 0,20 |
| NaOH (45%-ig) | 0,20 |
| Konservierungsmittel | q.s. |
| Parfum | q.s. |
| Wasser, demin. | ad 100,00 |

| Beispiel 3<br>O/W-Lotion | |
|---|---|
| Glycerylstearat SE | 3,50 |
| Stearinsäure | 1,80 |
| Glycerin | 3,00 |
| Cetylstearylalkohol | 0,50 |
| Octyldodecanol | 7,00 |
| Dicaprylylether | 8,00 |

(fortgesetzt)

| Beispiel 3<br>O/W-Lotion | |
|---|---|
| Cetylstearylisononanoat | 6,00 |
| Pathionic® ISL | 1,00 |
| hydrophobes $TiO_2$ | 1,00 |
| Carbomer | 0,20 |
| NaOH (45%-ig) | 0,20 |
| Konservierungsmittel | q.s. |
| Parfum | q.s. |
| Wasser, demin. | ad 100,00 |

| Beispiel 4<br>O/W-Lotion | |
|---|---|
| Glycerylstearat SE | 3,50 |
| Stearinsäure | 1,80 |
| Glycerin | 3,00 |
| Cetylstearylalkohol | 0,50 |
| Octyldodecanol | 7,00 |
| Dicaprylylether | 8,00 |
| Cetylstearylisononanoat | 6,00 |
| Pathionic® ISL | 1,00 |
| hydrophobes ZnO | 1,00 |
| Carbomer | 0,20 |
| NaOH (45%-ig) | 0,20 |
| Konservierungsmittel | q.s. |
| Parfum | q.s. |
| Wasser, demin. | ad 100,00 |

| Beispiel 5<br>O/W-Lotion | |
|---|---|
| Glycerylstearat SE | 3,50 |
| Stearinsäure | 1,80 |
| Glycerin | 3,00 |
| Cetylstearylalkohol | 0,50 |

(fortgesetzt)

| Beispiel 5<br>O/W-Lotion | |
|---|---|
| Octyldodecanol | 7,00 |
| Dicaprylylether | 8,00 |
| Cetylstearylisononanoat | 6,00 |
| Miranol® C32 | 1,00 |
| hydrophobes $TiO_2$ | 1,00 |
| Carbomer | 0,20 |
| NaOH (45%-ig) | 0,20 |
| Konservierungsmittel | q.s. |
| Parfum | q.s. |
| Wasser, demin. | ad 100,00 |

| Beispiel 6<br>O/W-Lotion | |
|---|---|
| Glycerylstearat SE | 3,50 |
| Stearinsäure | 1,80 |
| Glycerin | 3,00 |
| Cetylstearylalkohol | 0,50 |
| Octyldodecanol | 7,00 |
| Dicaprylylether | 8,00 |
| Cetylstearylisononanoat | 6,00 |
| Pathionic® ISL | 1,00 |
| hydrophobes ZnO | 1,00 |
| Carbomer | 0,20 |
| NaOH (45%-ig) | 0,20 |
| Konservierungsmittel | q.s. |
| Parfum | q.s. |
| Wasser, demin. | ad 100,00 |

| Beispiel 7<br>O/W-Lotion | |
|---|---|
| Glycerylstearat SE | 3,50 |
| Emulgade®F | 3,50 |
| Glycerin | 3,00 |

(fortgesetzt)

| Beispiel 7<br>O/W-Lotion | |
|---|---|
| Cetylstearylalkohol | 1,50 |
| Octyldodecanol | 7,00 |
| Capryl-/Caprinsäuretriglycerid | 5,00 |
| Cetylstearylisononanoat | 6,00 |
| Uvinul®T150 | 2,50 |
| Plantaren® 2000 | 1,00 |
| hydrophobes $TiO_2$ | 1,00 |
| Carbomer | 0,20 |
| NaOH (45%-ig) | 0,13 |
| Konservierungsmittel | q.s. |
| Parfum | q.s. |
| Wasser, demin. | ad 100,00 |

| Beispiel 8<br>O/W-Lotion | |
|---|---|
| Glycerylstearat SE | 3,50 |
| Emulgade®F | 3,50 |
| Glycerin | 3,00 |
| Cetylstearylalkohol | 1,50 |
| Octyldodecanol | 7,00 |
| Capryl-/Caprinsäuretriglycerid | 5,00 |
| Cetylstearylisononanoat | 6,00 |
| Uvinul®T150 | |
| Plantaren® 2000 | 1,00 |
| hydrophobes $ZnO_2$ | 1,00 |
| Carbomer | 0,20 |
| NaOH (45%-ig) | 0,13 |
| Konservierungsmittel | q.s. |
| Parfum | q.s. |
| Wasser, demin. | ad 100,00 |

| Beispiel 9<br>O/W-Lotion | |
|---|---|
| Glycerylstearat SE | 3,50 |
| Emulgade®F | 3,50 |
| Glycerin | 3,00 |
| Cetylstearylalkohol | 1,50 |
| Octyldodecanol | 7,00 |
| Capryl-/Caprinsäuretriglycerid | 5,00 |
| Cetylstearylisononanoat | 6,00 |
| Uvinul®T150 | |
| Pathionic® ISL | 1,00 |
| hydrophobes $TiO_2$ | 1,00 |
| Carbomer | 0,20 |
| NaOH (45%-ig) | 0,13 |
| Konservierungsmittel | q.s. |
| Parfum | q.s. |
| Wasser, demin. | ad 100,00 |

| Beispiel 10<br>O/W-Lotion | |
|---|---|
| Glycerylstearat SE | 3,50 |
| Emulgade®F | 3,50 |
| Glycerin | 3,00 |
| Cetylstearylalkohol | 1,50 |
| Octyldodecanol | 7,00 |
| Capryl-/Caprinsäuretriglycerid | 5,00 |
| Cetylstearylisononanoat | 6,00 |
| Uvinul®T150 | |
| Pathionic® ISL | 1,00 |
| hydrophobes $ZnO_2$ | 1,00 |
| Carbomer | 0,20 |
| NaOH (45%-ig) | 0,13 |
| Konservierungsmittel | q.s. |
| Parfum | q.s. |
| Wasser, demin. | ad 100,00 |

| Beispiel 11<br>O/W-Lotion | |
|---|---|
| Glycerylstearat SE | 3,50 |
| Emulgade®F | 3,50 |
| Glycerin | 3,00 |
| Cetylstearylalkohol | 1,50 |
| Octyldodecanol | 7,00 |
| Capryl-/Caprinsäuretriglycerid | 5,00 |
| Cetylstearylisononanoat | 6,00 |
| Uvinul®T150 | |
| Miranol® C32 | 1,00 |
| hydrophobes $TiO_2$ | 1,00 |
| Carbomer | 0,20 |
| NaOH (45%-ig) | 0,13 |
| Konservierungsmittel | q.s. |
| Parfum | q.s. |
| Wasser, demin. | ad 100,00 |

| Beispiel 12<br>O/W-Lotion | |
|---|---|
| Glycerylstearat SE | 3,50 |
| Emulgade®F | 3,50 |
| Glycerin | 3,00 |
| Cetylstearylalkohol | 1,50 |
| Octyldodecanol | 7,00 |
| Capryl-/Caprinsäuretriglycerid | 5,00 |
| Cetylstearylisononanoat | 6,00 |
| Uvinul®T150 | |
| Miranol® C32 | 1,00 |
| hydrophobes $ZnO_2$ | 1,00 |
| Carbomer | 0,20 |
| NaOH (45%-ig) | 0,13 |
| Konservierungsmittel | q.s. |
| Parfum | q.s. |
| Wasser, demin. | ad 100,00 |

| Beispiel 13 | |
|---|---|
| Methylglucosesesquistearat | 3,50 |
| Butylenglykol | 5,00 |
| Cetylstearylalkohol | 3,00 |
| Cetylstearylisononanoat | 6,00 |
| Xanthan Gum | 0,20 |
| $C_{12}$-$C_{15}$ Alkylbenzoate | 10,0 |
| Uvinul® T150 | 4,00 |
| Plantaren® 2000 | 1,00 |
| hydrophobes $TiO_2$ | 4,00 |
| Konservierungsmittel | q.s. |
| Parfum | q.s. |
| Wasser, demin. | ad 100,00 |

| Beispiel 14 | |
|---|---|
| Methylglucosesesquistearat | 3,50 |
| Butylenglykol | 5,00 |
| Cetylstearylalkohol | 3,00 |
| Cetylstearylisononanoat | 6,00 |
| Xanthan Gum | 0,20 |
| $C_{12}$-$C_{15}$ Alkylbenzoate | 10,0 |
| Uvinul® T150 | 4,00 |
| Plantaren® 2000 | 1,00 |
| hydrophobes ZnO | 4,00 |
| Konservierungsmittel | q.s. |
| Parfum | q.s. |
| Wasser, demin. | ad 100,00 |

| Beispiel 15 | |
|---|---|
| Methylglucosesesquistearat | 3,50 |
| Butylenglykol | 5,00 |
| Cetylstearylalkohol | 3,00 |

(fortgesetzt)

| Beispiel 15 | |
|---|---|
| Cetylstearylisononanoat | 6,00 |
| Xanthan Gum | 0,20 |
| $C_{12}$-$C_{15}$ Alkylbenzoate | 10,0 |
| Uvinul®T150 | 4,00 |
| Pathionic® ISL | 1,00 |
| hydrophobes $TiO_2$ | 4,00 |
| Konservierungsmittel | q.s. |
| Parfum | q.s. |
| Wasser, demin. | ad 100,00 |

| Beispiel 16 | |
|---|---|
| Methylglucosesesquistearat | 3,50 |
| Butylenglykol | 5,00 |
| Cetylstearylalkohol | 3,00 |
| Cetylstearylisononanoat | 6,00 |
| Xanthan Gum | 0,20 |
| $C_{12}$-$C_{15}$ Alkylbenzoate | 10,0 |
| Uvinul®T150 | 4,00 |
| Pathionic® ISL | 1,00 |
| hydrophobes ZnO | 4,00 |
| Konservierungsmittel | q.s. |
| Parfum | q.s. |
| Wasser, demin. | ad 100,00 |

| Beispiel 17 | |
|---|---|
| Methylglucosesesquistearat | 3,50 |
| Butylenglykol | 5,00 |
| Cetylstearylalkohol | 3,00 |
| Cetylstearylisononanoat | 6,00 |
| Xanthan Gum | 0,20 |
| $C_{12}$-$C_{15}$ Alkylbenzoate | 10,0 |
| Uvinul®T150 | 4,00 |
| Miranol® C32 | 1,00 |

(fortgesetzt)

| Beispiel 17 | |
|---|---|
| hydrophobes $TiO_2$ | 4,00 |
| Konservierungsmittel | q.s. |
| Parfum | q.s. |
| Wasser, demin. | ad 100,00 |

| Beispiel 18 | |
|---|---|
| Methylglucosesesquistearat | 3,50 |
| Butylenglykol | 5,00 |
| Cetylstearylalkohol | 3,00 |
| Cetylstearylisononanoat | 6,00 |
| Xanthan Gum | 0,20 |
| $C_{12}$-$C_{15}$ Alkylbenzoate | 10,0 |
| Miranol® C32 | 1,00 |
| hydrophobes ZnO | 4,00 |
| Konservierungsmittel | q.s. |
| Parfum | q.s. |
| Wasser, demin. | ad 100,00 |

| Beispiel 19<br>O/W-Lotion | |
|---|---|
| | Gew.-% |
| Glycerylstearat | 3,50 |
| Stearinsäure | 1,80 |
| Glycerin | 3,00 |
| Tego®Betain 810 | 0,50 |
| Natriumhydroxid (45%ig) | 0,20 |
| Octyldodecanol | 7,00 |
| Dicaprylylether | 8,00 |
| Titandioxid T805 | 4,00 |
| Carbomer | 0,20 |
| Konservierungsmittel | q.s. |
| Parfum | q.s. |
| Wasser, demin. | ad 100,00 |

| Beispiel 20<br>Hydrodispersionsgel | |
|---|---|
| | Gew.-% |
| Pemulen TR-1 | 0,50 |
| Ethanol | 3,50 |
| Glycerin | 3,00 |
| Dimethicon | 1,50 |
| Natriumhydroxid (45%ig) | 0,55 |
| Octyldodecanol | 0,50 |
| Capric/Caprylic Triglyceride | 5,00 |
| Titandioxid T805 | 2,00 |
| Carbomer | 0,20 |
| Konservierungsmittel | q.s. |
| Parfum | q.s. |
| Wasser, demin. | ad 100,00 |

| Beispiel 21<br>O/W-Creme | |
|---|---|
| | Gew.-% |
| Glycerylstearat SE | 3,50 |
| Stearinsäure | 3,50 |
| Butylenglykol | 5,00 |
| Cetylstearylalkohol | 3,00 |
| Natriumhydroxid (45%ig) | 0,35 |
| $C_{12}$-$C_{15}$ Alkylbenzoate | 10,0 |
| Tego®Betain 810 | 0,50 |
| Titandioxid T805 | 2,00 |
| Carbomer | 0,20 |
| Konservierungsmittel | q.s. |
| Parfum | q.s. |
| Wasser, demin. | ad 100,00 |

| Beispiel 22<br>O/W-Lotion | |
|---|---|
| | Gew.-% |
| Glycerylstearat | 3,50 |
| Arlacel 60 | 1,80 |
| Glycerin | 3,00 |
| Tego®Betain 810 | 0,50 |
| Natriumhydroxid (45%ig) | 0,20 |
| Octyldodecanol | 7,00 |
| Dicaprylylether | 8,00 |
| Zinkoxid Neutral | 4,00 |
| Carbomer | 0,20 |
| Konservierungsmittel | q.s. |
| Parfum | q.s. |
| Wasser, demin. | ad 100,00 |

| Beispiel 23<br>Hydrodispersionsgel | |
|---|---|
| | Gew.-% |
| Pemulen TR-1 | 0,50 |
| Ethanol | 3,50 |
| Glycerin | 3,00 |
| Dimethicon | 1,50 |
| Natriumhydroxid (45%ig) | 0,55 |
| Octyldodecanol | 0,50 |
| Capric/Caprylic Triglyceride | 5,00 |
| Zinkoxid Neutral | 2,00 |
| Carbomer | 0,20 |
| Konservierungsmittel | q.s. |
| Parfum | q.s. |
| Wasser, demin. | ad 100,00 |

| Beispiel 24<br>O/W-Creme | |
|---|---|
| | Gew.-% |
| Arlacel 60 | 3,50 |
| Behenylalkohol | 3,00 |
| Butylenglykol | 5,00 |
| Cetylstearylalkohol | 3,50 |
| Natriumhydroxid (45%ig) | 0,35 |
| $C_{12}$-$C_{15}$ Alkylbenzoate | 10,0 |
| Tego®Betain 810 | 0,25 |
| Zinkoxid Neutral | 2,00 |
| Carbomer | 0,20 |
| Konservierungsmittel | q.s. |
| Parfum | q.s. |
| Wasser, demin. | ad 100,00 |

## Patentansprüche

1. O/W-Emulsionen,

   - enthaltend eine Wasserphase, gegebenenfalls übliche, in Wasser lösliche oder dispergierbare Substanzen,
   - enthaltend eine Ölphase, in welcher ein oder mehrere anorganische Pigmente, insbesondere hydrophobe anorganische Mikropigmente, in suspendierter Form vorliegen,
   - enthaltend mindestens ein hydrophiles Tensid.

2. Verwendung von hydophilen Tensiden in O/W-Emulsionen, diese

   - enthaltend eine Wasserphase, gegebenenfalls übliche, in Wasser lösliche oder dispergierbare Substanzen,
   - enthaltend eine Ölphase, in welcher ein oder mehrere anorganische Pigmente, insbesondere hydrophobe anorganische Mikropigmente, in suspendierter Form vorliegen,

   zur Stabilisierung der O/W-Emulsionen gegen die Migration der anorganischen Pigmente, insbesondere hydrophoben anorganischen Mikropigmente aus der Ölphase in die Wasserphase und die Folgen einer solchen Migration.

3. Verwendung von hydophilen Tensiden in O/W-Emulsionen, diese

   - enthaltend eine Wasserphase, gegebenenfalls übliche, in Wasser lösliche oder dispergierbare Substanzen,
   - enthaltend eine Ölphase, in welcher ein oder mehrere anorganische Pigmente, insbesondere hydrophobe anorganische Mikropigmente, in suspendierter Form vorliegen,

   zur Stabilisierung der O/W-Emulsionen gegen das Ausweißeln der anorganischen Pigmente, insbesondere hydrophoben anorganischen Mikropigmente.

4. O/W-Emulsionen nach Anspruch 1 oder Verwendung nach Anspruch 2 oder 3, dadurch gekennzeichnet, daß die hydrophilen Tenside gewählt werden aus der Gruppe der Alkylglucoside, der Acyllactylate, der Betaine sowie der Cocoamphoacetate.

5. O/W-Emulsionen nach Anspruch 4 oder Verwendungen nach Anspruch 4, dadurch gekennzeichnet, daß die

Alkylglucoside gewählt werden aus der Gruppe der Alkylglucoside, welche sich durch die Strukturformel

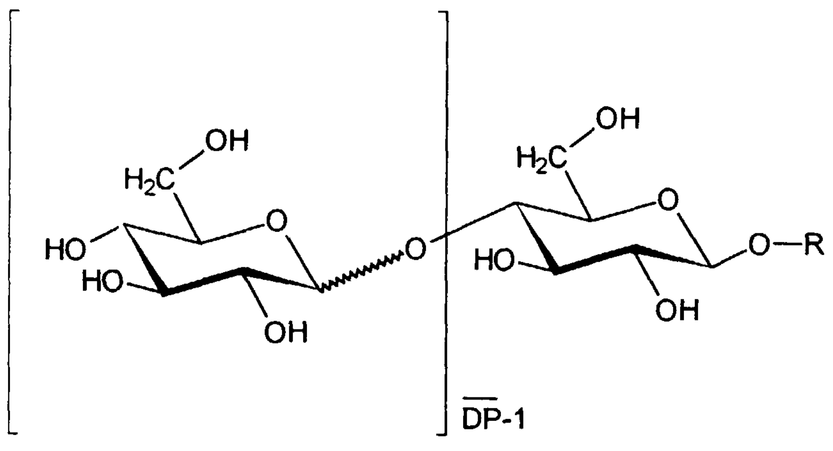

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

**6.** O/W-Emulsionen oder Verwendungen nach Anspruch 5, dadurch gekennzeichnet, daß das oder die Alkylglucoside gewählt werden aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

**7.** O/W-Emulsionen oder Verwendungen nach Anspruch 4, dadurch gekennzeichnet, daß das oder die Acyllactylate gewählt werden aus der Gruppe der Substanzen, welche sich durch die Strukturformel

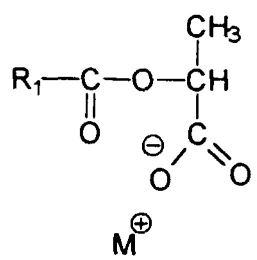

auszeichnen, wobei $R_1$ einen verzweigten oder unverzweigten Alkylrest mit 1 bis 30 Kohlenstoffatomen und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

**8.** O/W-Emulsionen oder Verwendungen nach Anspruch 4, dadurch gekennzeichnet, daß das oder die Betaine gewählt werden aus der Gruppe der Substanzen, welche sich durch die Strukturformel

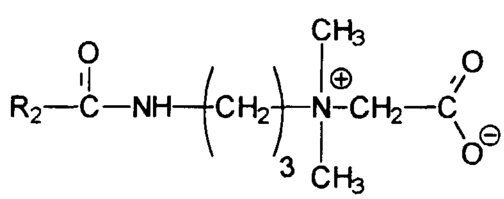

auszeichnen, wobei $R_2$ einen verzweigten oder unverzweigten Alkylrest mit 1 bis 30 Kohlenstoffatomen bedeutet.

**9.** O/W-Emulsionen oder Verwendungen nach Anspruch 8, dadurch gekennzeichnet, daß $R_2$ einen verzweigten oder unverzweigten Alkylrest mit 6 bis 12 Kohlenstoffatomen bedeutet.

**10.** O/W-Emulsionen oder Verwendungen nach Anspruch 4, dadurch gekennzeichnet, daß als Cocoamphoacetat das Natriumcocoamphoacetat gewählt wird.

**11.** O/W-Emulsionen oder Verwendungen nach Anspruch 4, dadurch gekennzeichnet, daß das oder die anorganischen Pigmente gewählt werden aus der Gruppe der oberflächlich hydrophobierten Metalloxide und/oder anderen in Wasser schwerlöslichen oder unlöslichen Metallverbindungen, insbesondere der Oxide des Titans ($TiO_2$), Zinks ($ZnO$), Eisens (z.B. $Fe_2O_3$), Zirkoniums ($ZrO_2$), Siliciums ($SiO_2$), Mangans (z.B. $MnO$), Aluminiums ($Al_2O_3$), Cers (z.B. $Ce_2O_3$), Mischoxiden der entsprechenden Metalle sowie Abmischungen aus solchen Oxiden.

Europäisches Patentamt

# EUROPÄISCHER RECHERCHENBERICHT

Nummer der Anmeldung
EP 98 10 9941

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP 0 628 303 A (TIOXIDE SPECIALTIES) 14. Dezember 1994<br>* Ansprüche 1,7,8,10 *<br>* Seite 2, Zeile 28-29 *<br>* Seite 3, Zeile 53-55 *<br>--- | 1,4,11 | A61K7/42 |
| X | EP 0 559 319 A (TIOXIDE SPECIALTIES) 8. September 1993<br>* Ansprüche 1,2,11 *<br>* Seite 2, Zeile 54-57 *<br>--- | 1,4,11 | |
| X | DE 195 32 543 A (HENKEL) 6. März 1997<br>* Ansprüche 1,5 *<br>* Seite 2, Zeile 67 - Seite 3, Zeile 2 *<br>* Seite 4, Zeile 27-29 *<br>* Seite 5, Zeile 14-26 *<br>* Seite 5, Zeile 52-54 *<br>--- | 1,4-6,11 | |
| X | DE 195 45 789 A (BEIERSDORF) 12. Juni 1997<br>* Ansprüche 1,4,5 *<br>* Seite 2, Zeile 31-33 *<br>* Seite 2, Zeile 66 - Seite 3, Zeile 10 *<br>--- | 1-3,11 | RECHERCHIERTE SACHGEBIETE (Int.Cl.6)<br>A61K |
| X | WO 90 11067 A (THE BOOTS COMPANY PLC) 4. Oktober 1990<br>* Ansprüche 1,8 *<br>* Seite 6, Zeile 12 - Seite 7, Zeile 28 *<br>--- | 1,11 | |
| X | DE 44 12 081 A (IFAC) 12. Oktober 1995<br>* Anspruch 1 *<br>* Seite 1, Zeile 45-51 *<br>* Beispiel 6 *<br>--- | 1,4,7 | |
| X | US 5 618 522 A (J.E.KALETA) 8. April 1997<br>* Ansprüche 1,8,10,11 *<br>* Spalte 11, Zeile 48-64 *<br>---<br>-/-- | 1,4,7 | |

Der vorliegende Recherchenbericht wurde für alle Patentansprüche erstellt

| Recherchenort | Abschlußdatum der Recherche | Prüfer |
|---|---|---|
| DEN HAAG | 12. Oktober 1998 | Peeters, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 98 10 9941

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US 5 547 659 A (M.A.RINALDI, L.R.ROBINSON) 20. August 1996<br>* Anspruch 1 *<br>* Spalte 2, Zeile 46-61 *<br>--- | 1-3,7 | |
| X | US 5 207 998 A (L.R.ROBINSON, M.A.RINALDI, A.J.GUPTE) 4. Mai 1993<br>* Ansprüche 1,5 *<br>* Spalte 2, Zeile 49-52 *<br>* Spalte 8, Zeile 33-48 *<br>--- | 1-4,11 | |
| P,X | US 5 747 012 A (G.H.DAHMS) 5. Mai 1998<br>* Ansprüche 1,2,12,18 *<br>----- | 1,4,11 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| DEN HAAG | 12. Oktober 1998 | Peeters, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 98 10 9941

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-10-1998

| Im Recherchenbericht angeführtes Patentdokument | Datum der Veröffentlichung | Mitglied(er) der Patentfamilie | Datum der Veröffentlichung |
|---|---|---|---|
| EP 628303 A | 14-12-1994 | AT 158169 T | 15-10-1997 |
| | | AU 675834 B | 20-02-1997 |
| | | AU 6314194 A | 15-12-1994 |
| | | CA 2123717 A | 12-12-1994 |
| | | DE 69405649 D | 23-10-1997 |
| | | DE 69405649 T | 22-01-1998 |
| | | ES 2107134 T | 16-11-1997 |
| | | GB 2279007 A,B | 21-12-1994 |
| | | JP 7145029 A | 06-06-1995 |
| | | ZA 9404023 A | 06-02-1995 |
| EP 559319 A | 08-09-1993 | AT 170743 T | 15-09-1998 |
| | | AU 658967 B | 04-05-1995 |
| | | AU 3205593 A | 02-09-1993 |
| | | CA 2088300 A | 30-08-1993 |
| | | GB 2264703 A,B | 08-09-1993 |
| | | JP 6039271 A | 15-02-1994 |
| | | US 5443759 A | 22-08-1995 |
| | | US 5516457 A | 14-05-1996 |
| | | ZA 9300893 A | 13-09-1993 |
| DE 19532543 A | 06-03-1997 | KEINE | |
| DE 19545789 A | 12-06-1997 | WO 9721421 A | 19-06-1997 |
| | | EP 0868168 A | 07-10-1998 |
| WO 9011067 A | 04-10-1990 | AU 625921 B | 16-07-1992 |
| | | AU 5265790 A | 22-10-1990 |
| | | DE 69002454 T | 18-11-1993 |
| | | DK 463030 T | 18-10-1993 |
| | | EP 0463030 A | 02-01-1992 |
| | | ES 2058897 T | 01-11-1994 |
| | | JP 4504116 T | 23-07-1992 |
| DE 4412081 A | 12-10-1995 | JP 7284645 A | 31-10-1995 |
| | | US 5674475 A | 07-10-1997 |
| US 5618522 A | 08-04-1997 | KEINE | |
| US 5547659 A | 20-08-1996 | AU 1602692 A | 12-11-1992 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 98 10 9941

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-10-1998

| Im Recherchenbericht angeführtes Patentdokument | Datum der Veröffentlichung | Mitglied(er) der Patentfamilie | Datum der Veröffentlichung |
|---|---|---|---|
| US 5547659 A | | AU 674634 B | 02-01-1997 |
| | | AU 3792695 A | 15-02-1996 |
| | | JP 5194171 A | 03-08-1993 |
| | | NZ 242624 A | 22-12-1994 |
| US 5207998 A | 04-05-1993 | AU 1602592 A | 12-11-1992 |
| | | AU 673790 B | 21-11-1996 |
| | | AU 3775695 A | 25-01-1996 |
| | | JP 5148127 A | 15-06-1993 |
| | | NZ 242623 A | 22-12-1994 |
| | | US 5306485 A | 26-04-1994 |
| US 5747012 A | 05-05-1998 | KEINE | |

EPO FORM P0461

Für nähere Einzelheiten zu diesem Anhang : siehe Amtsblatt des Europäischen Patentamts, Nr.12/82